# EUROPEAN PATENT SPECIFICATION

(11) **EP 1 974 030 B1**
(45) Date of publication and mention of the grant of the patent: **14.09.2016**
(21) Application number: 07711341.3
(22) Date of filing: 12.01.2007
(51) Int. Cl.: A23K 40/30, A23K 20/189, A23K 20/158, C12N 11/02, C12N 9/16

(54) **IMPROVED ENZYME FORMULATIONS FOR ANIMAL FEED**
VERBESSERTE ENZYMFORMULIERUNGEN FÜR TIERFUTTER
FORMULATIONS ENZYMATIQUES AMELIOREES POUR ALIMENT ANIMAL

(30) Priority: 17.01.2006 GB 0600913
(43) Date of publication of application: 01.10.2008
(73) Proprietor: AB Enzymes GmbH, 64293 Darmstadt (DE)
(72) Inventor: WINN, Peter, David, Berkshire RG42 6EY (GB); SILVERTHORNE, John, Martin, Berks RG42 6EY (GB); STREET, Peter, Francis, Scott, Wiltshire, SN8 3BL (GB); MULQUEEN, Patrick, Joseph, Berkshire RG42 6EY (GB)
(74) Representative: Hiebl, Inge Elisabeth
(86) International application number: PCT/EP2007/000263
(87) International publication number: WO 2007/082693

(56) References cited:
- WO-A-92/12645
- WO-A-94/17106
- WO-A-98/27199
- US-A- 6 136 772
- US-A1- 2005 163 765
- US-B1- 6 500 426
- US-B1- 6 610 519

### SEQUENCE LISTING

<110> AB Enzymes GmbH
<120> Improved Enzyme Formulations for Animal Feed
<130> 18551EP
<140> EP 07 711 341.3
   <141> 2007-01-12
<150> GB 20060000913
   <151> 2006-01-17
<160> 1
<170> PatentIn version 3.3
<210> 1
   <211> 410
   <212> PRT
   <213> Artificial sequence
<220>
   <223> Engineered phytase
<400> 1
   Gln Ser Glu Pro Glu Leu Lys Leu Glu Ser Val Val Ile Val Ser Arg
   1               5                   10                  15
   His Gly Val Arg Ala Pro Thr Lys Ala Thr Gln Leu Met Gln Asp Val
               20                  25                  30
   Thr Pro Asp Ala Trp Pro Thr Trp Pro Val Lys Leu Gly Glu Leu Thr
           35                  40                  45
   Pro Arg Gly Gly Glu Leu Ile Ala Tyr Leu Gly His Tyr Trp Arg Gln
       50                  55                  60
   Arg Leu Val Ala Asp Gly Leu Leu Pro Lys Cys Gly Cys Pro Gln Ser
   65                  70                  75                  80
   Gly Gln Val Ala Ile Ile Ala Asp Val Asp Glu Arg Thr Arg Lys Thr
                   85                  90                  95
   Gly Glu Ala Phe Ala Ala Gly Leu Ala Pro Asp Cys Ala Ile Thr Val
               100                 105                 110
   His Thr Gln Ala Asp Thr Ser Ser Pro Asp Pro Leu Phe Asn Pro Leu
           115                 120                 125
   Lys Thr Gly Val Cys Gln Leu Asp Asn Ala Asn Val Thr Asp Ala Ile
       130                 135                 140
   Leu Glu Arg Ala Gly Gly Ser Ile Ala Asp Phe Thr Gly His Tyr Gln
   145                 150                 155                 160
   Thr Ala Phe Arg Glu Leu Glu Arg Val Leu Asn Phe Pro Gln Ser Asn
                   165                 170                 175
   Leu Cys Leu Lys Arg Glu Lys Gln Asp Glu Ser Cys Ser Leu Thr Gln
               180                 185                 190
   Ala Leu Pro Ser Glu Leu Lys Val Ser Ala Asp Cys Val Ser Leu Thr
           195                 200                 205
   Gly Ala Val Ser Leu Ala Ser Met Leu Thr Glu Ile Phe Leu Leu Gln
       210                 215                 220
   Gln Ala Gln Gly Met Pro Glu Pro Gly Trp Gly Arg Ile Thr Asp Ser
   225                 230                 235                 240
   His Gln Trp Asn Thr Leu Leu Ser Leu His Asn Ala Gln Phe Asp Leu
                   245                 250                 255
   Leu Gln Arg Thr Pro Glu Val Ala Arg Ser Arg Ala Thr Pro Leu Leu
               260                 265                 270
   Asp Leu Ile Lys Thr Ala Leu Thr Pro His Pro Pro Gln Lys Gln Ala
           275                 280                 285
   Tyr Gly Val Thr Leu Pro Thr Ser Val Leu Phe Ile Ala Gly His Asp
       290                 295                 300
   Thr Asn Leu Ala Asn Leu Gly Gly Ala Leu Glu Leu Asn Trp Thr Leu
   305                 310                 315                 320
   Pro Gly Gln Pro Asp Asn Thr Pro Pro Gly Gly Glu Leu Val Phe Glu
                   325                 330                 335
   Arg Trp Arg Arg Leu Ser Asp Asn Ser Gln Trp Ile Gln Val Ser Leu
               340                 345                 350
   Val Phe Gln Thr Leu Gln Gln Met Arg Asp Lys Thr Pro Leu Ser Leu
           355                 360                 365
   Asn Thr Pro Pro Gly Glu Val Lys Leu Thr Leu Ala Gly Cys Glu Glu
       370                 375                 380
   Arg Asn Ala Gln Gly Met Cys Ser Leu Ala Gly Phe Thr Gln Ile Val
   385                 390                 395                 400
   Asn Glu Ala Arg Ile Pro Ala Cys Ser Leu
                   405                 410

## Description

### FIELD OF THE INVENTION

The present invention relates to improvements in the manufacture of animal feeds. More particularly, the invention relates to methods for producing an improved enzyme containing product for use in the manufacture of animal feed and to the products produced by such methods.

### BACKGROUND OF THE INVENTION

Animal feeds are predominantly composed of cereals and vegetable proteins, a large portion of which cannot be fully digested by monogastric animals, including swine and poultry. Much of the energy available is locked up in the form of non-starch polysaccharides (NSP) that monogastric animals are unable to digest. Similarly, most plant materials used in animal feeds contain the mineral phosphorus which is bound in the form of phytic acid and cannot be degraded by monogastric animals. External enzymes, for example phytase, added directly to feeds act as supplements to the normal digestive enzymes already present in the animal's digestive system. The addition of enzymes helps to increase digestibility, facilitating better utilization of feeds. The more an animal can utilize the feed the better the animal performance (e.g., increased weight gain), and the lesser the load on the environment in terms of manure or other waste.

For the utilization of phytate phosphorus and minerals and trace elements bound in phytic acid complexes, hydrolysis of the ester-type bonded phosphate groups of phytic acid by phytase is necessary. Phytases belong to a special group of phosphatases which are capable of hydrolyzing phytate to a series of lower phosphate esters of myo-inositol and phosphate. Two types of phytases are known: 3-phytase and 6-phytase, indicating the initial attack of the susceptible phosphate ester bond. Although monogastric animals lack sufficient phytase to effectively utilize phytate phosphorous, many fungi, bacteria and yeasts produce phytase that can be used to supplement animal rations.

The beneficial effects of supplementary phytases on phosphorus digestibility and animal performance have been well documented. The efficacy of any enzyme preparation depends not only on the type, inclusion rate and level of activity present, but also on the ability of the enzyme to maintain its activity in the different conditions encountered through the gastrointestinal tract and the conditions used for the pre-treatment of a feed formulation.

Unfortunately, during the feed manufacturing process (generally extrusion, pelleting, etc.), high temperature and pressure conditions often result in a significant loss of valuable heat sensitive and/or water-soluble ingredients such as enzymes. Ingredients may also be lost in post-manufacturing processes when feeds are exposed to air and water during, for example, storage and handling, or from conditions that occur in the animal's own system. The loss of ingredient value or functionality can be costly and increase the risk of missing a targeted feed composition that is necessary for optimal performance of the animal.

Because most currently available commercial enzymes are not thermotolerant, they are often applied post pelleting, generally via spraying an enzyme solution onto pelleted feed to avoid heat inactivation of the enzyme(s) which would occur during the pelleting process. However the amounts of enzyme in the final feed preparations are usually very small which makes it difficult to achieve a homogenous distribution of the enzyme in the feed, and liquids are notoriously more difficult to mix evenly than dry ingredients. In addition one needs specialized (expensive) equipment to add liquids to the feed after pelleting which is not currently available at most feed mills (due to the extra cost).

Dry formulations of enzyme(s), on the other hand, have the potential disadvantage of heat-inactivation of the enzymes during pelleting. Preferred manufacturing protocols in the feed industry involve steam pelleting where the feed is subjected to steam injection(s) prior to pelleting. In the subsequent pelleting step the feed is forced through a matrix or die and the resulting extrudate strips are cut into suitable pellets of variable length. The moisture content immediately before pelleting is generally between 15-25% and typically between 18% and 19%. During this process the feed temperatures may rise to 60-95°C or higher. The combined effect of high moisture content and high temperature is detrimental to many enzymes. These disadvantages are also encountered in other types of thermomechanical treatments such as extrusion and expansion.

One approach to solving the problem of loss of enzyme activity during the heating process has been to discover or bioengineer thermotolerant enzymes. For example, US Patent 7,135,323 discloses a bioengineered thermotolerant phytase which retains at least 40% activity after 30 minutes at about 60°C, and which has a high specific activity, i.e., at least about 200 phytase units/mg at 37°C and at acid pH, e.g., pH 4.5. Such a thermotolerant phytase commercially available as Quantum™ Phytase has an 8-fold higher specific activity than other commercially available
enzymes, and may be added prior to pelleting, thereby facilitating production of a feed with an improved distribution of the enzyme. Moreover, feed comprising the Quantum™ Phytase may have a longer shelf life than feed sprayed with phytase, as the spraying process introduces moisture which can support fungal and bacterial growth during storage.

There have been previous attempts to protect some of the more susceptible or costly ingredients by coating them with various materials. Approaches practiced to date include coating combinations of chemically altered ingredients with various materials to achieve protection. One technique used was to coat the ingredient with some form of lipid such as fats or fatty acids derived from a variety of animal and vegetable sources using a process such as microencapsulation. However, such coatings all have a common characteristic in that their melting points commonly do not exceed about 70°C. Therefore, their effective use as a protective coating is usually limited to processing environments below 70°C. The temperatures associated with extrusion and pelleting processes are typically greater than 70°C and feeds produced by extrusion often require drying at temperatures exceeding 100°C, thus rendering lipid coatings largely ineffective for maximum ingredient protection when using such high heat manufacturing processes. In addition, these techniques employ large quantities of lipid which is typically laid down in successive layers around the ingredient. This may greatly impair the bioavailability of the treated product.

US 2005/163765 discloses a process for the preparation of an enzyme-containing granulate, wherein an aqueous enzyme-containing liquid is, optionally supplemented with a solid carrier and/or additive ingredients, processed into granules, dried and subsequently coated with a polyolefin, preferably polypropylene and/or polyethylene.

WO 92/12645 discloses an enzyme containing T-granulate which is coated with a coating agent comprising a high melting fat or wax.

US 6,610,519 discloses a solid phytase composition having a phytase activity of above 20 FYT/g containing a lactic acid source such as corn steep liquor to stabilize the phytase.

US 6,500,426 discloses enzyme-containing granules for manufacturing animal feed compositions using a carrier containing at least about 15% (w/w) carbohydrate such as starch and less than 5% (w/w) protein.

US 6,136,772 discloses enzyme-containing granules comprising (a) an enzyme and (b) a core which intrinsically is capable of absorbing at least 5% w/w (based on the weight of the core) of water and processes for the production of such granules.

WO 94/17106 discloses a method and preparation for the storage and delivery of purified protein reagents which comprises an amount of a first wax carrier, the first wax carrier having a first melting point, and an amount of a first reagent, wherein the first reagent is a substantially purified preparation of a protein reagent.

WO 98/27199 discloses a process for the immobilization of an enzyme, comprising the steps: a. selecting an amphiphilic enzyme for immobilization, b. preparing an emulsion comprising a continuous hydrophobic phase and a dispersed aqueous phase which aqueous phase contains the enzyme and material suitable to act as carrier for the enzyme when the next step is carried out, which material is partly dissolved and partly not dissolved in the aqueous phase, c. removing water from the dispersed phase until this phase turns into solid enzyme coated particles.

Although thermotolerant enzymes such as phytase greatly improve the manufacture of animal feed, further improvements are needed in order to protect the enzyme from even higher temperatures yet maintain its bioavailability. Therefore, a need exists to provide an enzyme containing product, which is suitable for use in the production of animal feed that exhibits improved enzyme stability during thermal processing.

Accordingly, the present invention provides a method of preparing an improved thermostable enzyme product for use in manufacture of animal feed.

### SUMMARY OF THE INVENTION

The present invention is directed to a method for preparing a thermostable enzyme product for use in the manufacture of animal feed comprising:
a. combining (i) an enzyme, (ii) a solid carrier and (iii) a meltable hydrophobic substance selected from hydrogenated castor oil, a blend of hydrogenated and unhydrogenated vegetable oil, a microcrystalline wax, 12-hydroxystearic acid, hydrogenated palm kernel oil, hydrogenated palm oil, hydrogenated rapeseed oil, and high melting paraffin wax to provide a combined product;
b. optionally applying sufficient heat to the combined product of step (a) to allow the meltable hydrophobic substance to melt;
c. cooling the combined product to provide the thermostable enzyme product; and
d. optionally drying the product to a desired moisture content.

The amount of meltable hydrophobic substance used in the present invention may be insufficient to provide a contiguous coating around the enzyme to be protected. The bioavailability of the enzyme is therefore maintained. Adding such a meltable hydrophobic substance to an enzyme formulation surprisingly and unexpectedly improves the thermal stability of the enzyme product over similar products without the meltable hydrophobic substance. Without being bound by theory, it is hypothesized that the improved performance of products produced according to the present invention is due to a combination of the increased heat capacity of the meltable hydrophobic substance (thereby reducing the direct Impact of heat an enzymes during the feed pelleting process) and the hydrophobic nature of the treatment reducing the rate of moisture (steam) ingress to the location of the enzyme in the product during the feed pelleting process. The meltable hydrophobic substance according to the present invention is selected as defined above and includes hydrogenated castor oil (HCO), hydrogenated palm kernel oil (HPKO), hydrogenated palm oil (FHPO or Akoflake Palm 58 (AP)) or hydrogenated rapeseed oil (FHRO or Akoflake FSR (AFx, where x= F (flake) or M (melt))), a blend of hydrogenated and unhydrogenated vegetable oil (PB3), 12-hydroxystearic acid (12-HAS), microcrystalline wax such as Cerit HOT, and high-melting paraffin waxes such as Mekon White. This meltable hydrophobic substance as defined above can be a single component or derived from mixtures of products designed to produce a desired melting point.

The thermotolerant enzyme product obtained according to the invention may be added as a supplement to animal feed or to components of feed prior to or during feed processing. The thermotolerant enzyme product obtained according to the invention may be added to a mixture of feed components prior to and/or during heat (e.g., steam) conditioning in a pellet mill. Thus, the invention includes methods of making and using a thermotolerant enzyme product. The present invention is also generally directed to a method of preparing a feed supplement for monogastric animals. The method includes treating an enzyme with a protective amount of a biocompatible meltable hydrophobic substance.

According to one aspect, the present invention provides a thermostable enzyme product for use in the manufacture of animal feed comprising:
a. combining (i) an enzyme, (ii) a solid carrier and (iii) a meltable hydrophobic substance selected from hydrogenated castor oil, a blend of hydrogenated and unhydrogenated vegetable oil, a microcrystalline wax, 12-hydroxystearic acid, hydrogenated palm kernel oil, hydrogenated palm oil, hydrogenated rapeseed oil, and high melting paraffin wax to provide a combined product;
b. optionally applying sufficient heat to the combined product of step (a) to allow the meltable hydrophobic substance to melt;
c. cooling the combined product to provide the thermostable enzyme product; and
d. optionally drying the product to a desired moisture content.

In an aspect of this embodiment, it is possible to alter the order of addition and/or processing, such as preparing a dry enzyme product as a first stage by adding enzyme to a solid carrier and thereafter adding the meltable hydrophobic substance.

In one embodiment, the enzyme is phytase and the meltable hydrophobic substance is selected from the group consisting of hydrogenated castor oil, hydrogenated palm kernel oil, hydrogenated rapeseed oil, hydrogenated palm oil, a blend of hydrogenated and unhydrogenated vegetable oil, 12-hydroxystearic acid, Cerit HOT and Mekon White.

The foregoing and other aspects of the invention will become more apparent from the following detailed description.

### DESCRIPTION OF THE SEQUENCE IN THE SEQUENCE LISTING

SEQ ID NO: 1 is the amino acid sequence of a phytase useful as a supplement in animal feed.

### DETAILED DESCRIPTION OF THE INVENTION

Enzymes such as phytase are important as feed supplements for animals, particularly monogastric animals like poultry and swine, and it is desirable to provide the maximum amount of enzyme. Since most commercial enzyme products are not thermostable the amount of enzyme available for animals is limited by the high heat/steam process under which animal feed is manufactured. Meltable hydrophobic substances and method for their use described herein can provide greater heat and water protection of enzyme functionality during animal feed manufacturing processes and maintain bioavailability.

In one embodiment, the present invention encompasses a method for preparing a thermostable enzyme product for use in the manufacture of animal feed comprising:
a. combining (i) an enzyme, (ii) a solid carrier and (iii) a meltable hydrophobic substance selected from hydrogenated castor oil, a blend of hydrogenated and unhydrogenated vegetable oil, a microcrystalline wax, 12-hydroxystearic acid, hydrogenated palm kernel oil, hydrogenated palm oil, hydrogenated rapeseed oil, and high melting paraffin wax to provide a combined product;
b. optionally applying sufficient heat to the combined product of step (a) to allow the meltable hydrophobic substance to melt;
c. cooling the combined product to provide the thermostable enzyme product; and
d. optionally drying the product to a desired moisture content.

In one aspect of this embodiment, the meltable hydrophobic substance is added in step (a) as solid flakes or as a pre-melted molten liquid. The skilled person will recognize that if the meltable hydrophobic substance is added as a pre-melted molten liquid, step (b) may not be necessary.

The components referred to in step (a) may be combined in a single step or alternatively, in separate steps. For example, the enzyme may first be combined with the solid carrier and optionally water, optionally dried, and then the resulting enzyme/carrier combination combined with the meltable hydrophobic substance. In one embodiment, the enzyme is produced in a plant, including but not limited to corn or wheat, and that enzyme containing grain is ground to produce either a granulate or flour that comprises active enzyme in sufficient quantity to produce a biological effect product when stabilized by the method of the invention. In another embodiment, one or more of the steps are carried out in a fluidized bed apparatus.

In another embodiment, the solid carrier is an absorbent and/or adsorbent material. Examples of solid carriers that are suitable for use in the method of the present invention include, without limitation, plant sourced absorbents such as ground seed grains, for example, ground corn, ground wheat, wheat middlings, soybean meal, rice hulls, corn gluten feed, corn grits, distiller's dried grains and other carriers suitable or approved for use in animal feed. The solid carrier may also be, but not limited to, a mineral sourced absorbent, for example silica, diatomaceous earth or clay. In one aspect of this embodiment the solid carrier is ground wheat or corn. In another aspect, the solid carrier is wheat or corn flour.

By "meltable" hydrophobic substance it is meant a hydrophobic substance which is solid at the typical ambient storage temperature of a feed product but melts at a temperature above this. In one embodiment, the melting temperatures will range from 25°C to 120°C. The upper temperature is limited by the ability to melt the hydrophobic substance in the process and the stability of the enzyme at these elevated temperatures for the processing period. In one aspect of this embodiment, the hydrophobic substance has a melting point in the range 25-95°C. In another aspect of this embodiment, the hydrophobic substance has a melting point in the range 30-90°C. In yet another aspect of this embodiment, the hydrophobic substance has a melting point in the range 50-90°C. In still another aspect of this embodiment, the hydrophobic substance has a melting point in the range 60-80°C. In still another aspect of this embodiment, the hydrophobic substance has a melting point in the range of 82-86°C. In another aspect of this embodiment, the meltable hydrophobic substance has a melting point in the range of 66-69°C. In still another aspect of this embodiment, the meltable hydrophobic substance has a melting point in the range of 58-60°C. In yet another aspect of this embodiment, the hydrophobic substance has a melting point in the range of 38-46°C.

In one embodiment, the enzyme containing product of the present invention may comprise any suitable quantity of a meltable hydrophobic substance that protects the enzyme and maintains bioavailability. In one aspect of this embodiment, the enzyme containing product comprises 1-30% by weight of a meltable hydrophobic substance. In another aspect of this embodiment, the enzyme containing product comprises 5-20% by weight of a meltable hydrophobic substance. In another aspect of this embodiment, the enzyme containing product comprises at least 5% or more by weight, for example 7.5%, 10%, 20%, or 30% of a meltable hydrophobic substance. Without being bound by theory, it is thought that the treatment of the enzyme with a meltable hydrophobic substance protects the enzyme product matrix from the effect of temperature and moisture during the pelleting process. A sufficient concentration of a meltable hydrophobic substance is added to the matrix to effect the treatment and secure enhanced retention of activity of the enzyme, regardless of the concentration of enzyme present in the matrix.

Typically, phytase enzyme, wherein the phytase comprises the amino acid sequence of SEQ ID NO: 1 is formulated to contain 2500 phytase units/g product as a concentrated granular product. This product is then mixed (diluted) with suitable feed agents and compounded via a heating/pelleting process to produce an animal feed containing a prescribed amount of phytase enzyme. This amount of enzyme is determined by the feed needs of the target animal species, age and intended use (e.g. layers or broilers for chickens), and the nature of the enzyme. It is possible to produce a concentrated product according to the invention wherein the enzyme loading is much higher. For example, it is possible to produce a granular product containing up to at least 20000 phytase units/g. Upper limits of concentration are limited predominantly by the ability to dose such a concentrated product accurately into a large mass of animal feed and ensure the enzyme is evenly distributed throughout the feed mass before and after the animal feed pelleting process. With a typical phytase product which is formulated to contain 2500 phytase units/g, increasing the concentration of the product to 20000 phytase units/g would require only 12.5% of the weight of a concentrated product to be applied compared to the standard 2500 phytase units/g. For a 40000 phytase units/g product, only 6.25% of the concentrated product would need to be applied to deliver the same effect as a standard 2500 phytase units/g product. That concentrated product will still be stabilized by the relatively low (1-30%) levels of meltable hydrophobic substance as described herein. Moreover, the cost savings of employing more concentrated phytase containing products are self evident. Should dosing and distribution of such concentrated products be problematical, it is also possible to consider an economic alternative to processing all the enzyme at a lower concentration by considering a pre-dilution of the concentrated enzyme (for example at 20000 phytase units/g) with a suitable diluent to produce an acceptable feedstock for the final pelletization process of the complete animal feed. This would introduce cost savings by the ability to granulate a much reduced quantity of enzyme with the meltable hydrophobic solid. Clearly, there are options here for one skilled in the art to vary the concentration of enzyme in the granulate product to offer improved cost benefits without compromising the increased thermal tolerance of the product achieved by treatment with a meltable hydrophobic solid. Although the concentrations of enzyme mentioned are specific to phytase enzyme, the principles may equally be applied to other enzyme systems depending on their use rates and specific activities. It is also possible to consider much lower levels of enzyme contained within the concentrated product. Such products will also require loadings of hydrophobic meltable solid in the range 1-30%.

In another embodiment, the invention encompasses an enzyme containing solution comprising one or more enzymes suitable for use in an animal feed. In one aspect of this embodiment, an enzyme is selected from the group consisting of phytase, phosphatase, xylanase, cellulase, glucanase, mannanase, amylase, alpha-amylase, glucoamylase, peptidase, lipase, esterase, mannase, chitinase, β-1,3- and β-1,4-glucanase, glucose oxidase, catalase, galactosidase, glucosidase, hemicellulase, invertase, pectinase, pullulase and protease. The skilled person will recognize that enzymes and other thermolabile feed ingredients other than those specifically listed can be used in the present invention. In another aspect of this embodiment, the enzyme is a phytase. In yet another aspect of this embodiment, the phytase comprises the amino acid sequence of SEQ ID NO: 1. In another embodiment, the enzyme to be provided in the present invention is a solid product rather than a solution.

The skilled person will recognize that the present invention can be applied to protect other thermal process-labile components of animal feed concentrates. Such species can be selected from, but not limited to any of the following groups, individually or in combination: vitamins, such as vitamin A, B₁₂, D, D₃, E, riboflavin, niacin, choline, folic acid etc..; nucleic acids and nucleotides etc..., such as guanine, thymidine, cytosine, adenine etc...; amino acids, such as glycine, lysine, threonine, tryptophan, arginine, tyrosine, methionine etc..; micro-organisms, such as *Aspergillus niger, A. oryzae, Bacillus subtilis, B. licheniformis, Lactobacillus acidophilus, L. bulgaricus* etc...; medications and vaccines, such as chlortetracycline, erythromycin, oxytetracycline etc...; and flavour enhancers, such as sugars, spices, essential oils, synthetic flavourings etc...

The meltable hydrophobic substance-treated enzyme product of the invention is mixed with suitable feed agents and compounded via a heating/pelleting process to produce an animal feed containing a prescribed amount of phytase enzyme. This process involves mixing all the components together compressing them though an extruder with steam injection to act as a binder/pasteurizing method to produce suitable feed pellets for administration to target animals (such as, but not limited to, poultry or swine). During this process the temperatures of the feed (referred to as the "mash") can be raised to about 90°C or higher. At these temperatures, the enzyme may be deactivated rapidly. The product of this process is then assayed for recovery of enzyme (expressed as % recovered relative to the equivalent, non-processed mash used to prepare the pellets). The product of an original granulation process (whereby a solution of enzyme is added directly to a milled granular wheat) is shown in Table 2 (reference QP2500D) as a comparison. The products of this invention are referred to by the type of meltable hydrophobic substance, for example without limitation, HCO is an hydrogenated castor oil with a typical melting point range of 82-86°C, PB3 is a blend of hydrogenated and non-hydrogenated vegetable oils with a typical melting point range of 38-46°C, Akoflake Palm 58 or FHPO is a hydrogenated (fully hardened) palm oil with a typical melting point range of 58-60°C, HPKO is a hardened palm kernel oil with a typical melting point range of 41-44°C; Akoflake FSR or FHRO is a hydrogenated (fully hardened) rapeseed oil with a typical melting point range of 66-69°C. It will be recognize by the person skilled in the art that the actual melting point may vary depending on environmental or physical conditions under which the meltable hydrophobic substance is heated, or the source of the meltable hydrophobic substance.

The present invention further provides a product obtainable by a method of the present invention, a method for preparing an animal feed comprising combining a product obtainable by a method of the present invention with suitable animal feed ingredients and an animal feed so produced.

The skilled person in the art will recognize that the method of the present invention may be used in other animal feed formulation processes including but not limited to, spray dried products, wherein a liquid enzyme containing solution is atomized in a spray drying tower to form small droplets which during its way down the drying tower dries up to form an enzyme containing particulate material. Very small particles can be produced this way (Michael S. Showell (editor); Powdered detergents; Surfactant Science Series; 1998; vol. 71; page 140-142; Marcel Dekker); Layered products, wherein the enzyme is coated as a layer around a preformed core particle, wherein an enzyme containing solution is atomized, typically in a fluid bed apparatus wherein the preformed core particles are fluidized, and the enzyme containing solution adheres to the core particles and dries up to leave a layer of dry enzyme on the surface of the core particle. Particles of a desired size can be obtained this way if a useful core particle of the desired size can be found. This type of product is described in, for example, WO 97/23606. Another type of product is known wherein an absorbing core particle is applied, and rather than coating the enzyme as a layer around the core, the enzyme is absorbed onto and/or into the surface of the core. Such a process is described in WO 97/39116. Extrusion or pelletized products, wherein an enzyme containing paste is pressed to pellets or under pressure is extruded through a small opening and cut into particles which is subsequently dried. Prilled products, wherein an enzyme powder is suspended in molten wax and the suspension is sprayed, eg through a rotating disk atomizer, into a cooling chamber where the droplets quickly solidify (Michael S. Showell (editor); Powdered detergents; Surfactant Science Series; 1998; vol. 71; page 140-142; Marcel Dekker). Mixer granulation products, wherein an enzyme containing liquid is added to a dry powder composition of conventional granulating components. The liquid and the powder in a suitable proportion is mixed in and as the moisture of the liquid is absorbed in the dry powder, the components of the dry powder will start to adhere and agglomerate and particles will build up forming granules comprising the enzyme.

The invention will be further described by the following examples, which are not intended to limit the scope of the invention in any manner. The skilled person in the art will recognize that various modifications and variations can be made in the present invention without departing from the scope or spirit of the invention. For example, features illustrated or described as part of one embodiment can be used on another embodiment to yield still further embodiments. It is intended that the present invention covers such modifications and variations as falling within the scope of the appended claims and their equivalents.

### Example 1. Preparation of enzyme concentrate

A commercial enzyme concentrate Quantum™ Phytase 2500D (QP2500D)(Syngenta Animal Nutrition, Inc.), a dry formulation of the phytase witch a guaranteed minimum concentration of 2500 phytase units/g, was used in the following examples. QP2500D is typically made by spraying a concentrated formulated liquid Quantum™ Phytase, for example QP25000CL, which has a minimum concentration of 25000 phytase units/g onto ground wheat. The wheat carrier is dried to a moisture content in the range of 10-13% in a continuous process fluid bed dryer.

When making animal feed pellets from a mash comprising feed components and the dry formulated phytase concentrate, the mash is processed by passing it through an art recognized conditioner. The conditioner comprises a mixing tube which also serves to inject steam at approximately 120°C and 2 bar pressure. The residence time in the conditioner is approximately 30 seconds.

In order to improve the thermal stability, for example a reduction in loss of enzyme activity, methods were developed to treat the enzyme with a meltable hydrophobic substance to minimize the effect of steam and temperature yet maintain bioavilability.

Example 2. Addition of solid meltable hydrophobic substance to dry particulate material Initial experiments indicated that spraying a melted hydrophobic substance, for example, hydrogenated castor oil (HCO; typical melting point 82-86°C), onto QP2500D in a fluidized bed was not suitable for providing an improved thermostable product. This is due to the fact that a temperature of approximately 100°C was required to maintain the hydrophobic substance in a liquid state throughout spraying and dispersion onto the particles resulting in an unacceptable loss of enzyme activity. Adding an emulsifier did not improve the process.

A process was then developed whereby solid HCO flakes (a meltable hydrophobic substance) are mixed with a particulate material in a fluid bed dryer (FBD) held at ambient temperature. After adequate mixing, the inlet air temperature is increased until the HCO flakes melt. Heating of the device is then stopped and the resulting treated product is cooled to ambient temperature.

In a typical process, solid HCO flakes were added to QP2500D in a Strea-1™ fluid bed dryer with a thick glass walled vessel (Aeromatic-Fielder; Bubendorf, Switzerland) at ambient temperature. After adequate mixing, the inlet air temperature was increased to approximately 105°C, which is above the melting point of HCO (82-86°C), until the HCO flakes melted and treated the QP2500D particles. Heating of the device was then stopped and the product cooled to ambient temperature. Because the temperature of the glass wall was lower than the product, some treated product stuck to the glass wall. Therefore, the treated product was screened through a 2mm mesh sieve to return the particle size to that of the original QP2500D particulate material. For these initial development experiments, HCO was added to QP2500D at a rate to give a 20% by weight loading of wax treatment to the QP2500D particles. This process resulted in a free-flowing finished product that exhibited improved thermal stability properties during animal feed manufacture compared with a product in which the meltable hydrophobic substance had not been added.

A number of oils, hydrogenated oils and paraffinic waxes were evaluated during the trials. Some of the commercially available food/feed oils and hydrogenated oils had low melting points in the range of 30-50°C and these were discarded for being too soft under ambient or slightly elevated temperatures for use in this particular process although subsequently used in the process described in Example 8. Several other oils and hydrogenated oils listed in Table 1 had melting points in the range of 70-90°C and were initially selected for further experiments in this process.

**Table 1. Meltable hydrophobic substances evaluated.**

| Name | Abbreviation | Composition | Typical melting point range (°C) |
|---|---|---|---|
| Hydrogenated castor oil | HCO | hydrogenated form of oil from castor oil plant *Ricinus communis* | 82-86 |
| Cerit HOT | CH | microcrystalline wax | 79-85 |
| 12-hydroxystearic acid | 12-HSA | derived by hydrolysis from HCO | 76-78 |
| Hydrogenated palm kernel oil | HPKO | hydrogenated form of oil from kernels of the oil palm *Elaeis spp* | 41-44 |
| Fully-hardened rapeseed oil | FHRO | hydrogenated form of oil from the seeds of the rapeseed plant *Brassica napus* | 66-69 |
| Fully-hardened palm oil | FHPO | hydrogenated form of oil from the fruit of the oil palm *Elaeis spp* | 58-60 |
| PB3 | PB3 | proprietary blend of hydrogenated vegetable oil and vegetable oil | 38-46 |
| Akoflake Palm 58 | AP | fully-hardened palm oil | 58-60 |
| Akoflake FSR | AFF | fully-hardened rapeseed oil | 66-69 |
| Mekon white | MW | high melting paraffin wax | 88-92 |

### Example 3. Further fluid bed dryer process development

During the process described in Example 1 several changes in temperature occur (i.e., heating and cooling) in each step. In order to optimize the manufacturing process of a dry particulate material, a single-step process was developed whereby during the preparation of the meltable hydrophobic substance-treated product, the solid carrier, enzyme and meltable hydrophobic substance could be processed together with minimal temperature change.

Two methods were developed: Method 1. A solid carrier is sprayed with a desired amount of liquid enzyme solution in a fluid bed dryer. Excess moisture is removed by increasing the inlet temperature. A solid meltable hydrophobic substance is added and mixed with the other components. The inlet air temperature is then increased to melt the hydrophobic substance onto the solid carrier/enzyme particulate material.

In a typical process, 50g of a nominal 25,000 phytase units/g phytase concentrate was sprayed onto 500g of ground wheat in a Strea-1™ fluid bed dryer, and the temperature increased to approximately 50°C to yield a dry enzyme-coated particulate material of nominal 2,500 phytase units/g. Then 100g of solid HCO flakes was added and mixed with the particulate material. The inlet air temperature was then increased to approximately 105-110°C until the HCO flakes melted and treated the particles.

Method 2. A solid carrier and a solid meltable hydrophobic substance are mixed together in a fluid bed dryer at ambient temperature. A liquid enzyme concentrate is then sprayed onto to the mixture. The inlet air temperature is progressively heated to a level to remove excess moisture and then to melt the hydrophobic substance as in Method 1.

In a typical process, 500g of ground wheat and 100g of solid HCO flakes were mixed together in a Strea-1™ fluid bed dryer at ambient temperature. 50g of a nominal 25,000 phytase units/g phytase concentrate was then sprayed onto the resulting mixture. The inlet air temperature was then progressively increased to approximately 105-110°C which removes excess moisture, melted the HCO and treated the particles.

This method has an advantage over Method 1 in that it minimizes the number of separate steps required to achieve the desired product.

Both Method 1 and Method 2 may optionally incorporate an intermediate step wherein the inlet air temperature is raised, typically to 80°C, in order to remove excess water from the components prior to melting the HCO.

Both method 1 and method 2 result in a free-flowing product which physically resembles the product obtained in Example 1.

### Example 4. Process scale-up

Further trials were carried out to evaluate the developed methods disclosed in Example 2 on a pilot scale Glatt^{®} fluid bed dryer with a stainless steel walled vessel (Glatt Ingenieurtechnik GmbH, Weimar, Germany). The use of a stainless steel vessel instead of a glass vessel resulted in much higher temperature vessel walls and a resulting reduction in finished product sticking to the walls. These trials showed that it is possible to use a single-step method to make a product comprising from approximately 10% to about 20% HCO with virtually no lumps forming on a 2.5-3.0 kg scale.

During the process, the inlet temperature was increased from ambient temperature to approximately 105-110°C to melt the HCO. Again, an intermediate step involving an inlet temperature of typically 80°C can optionally be incorporated in order to remove excess moisture. The inlet temperature was typically achieved within a couple of minutes of increasing the inlet air temperature, but the outlet air temperature was observed to rise slowly over a period of typically 10 to 40 minutes, dependent on scale. As more water is driven off, this effect gradually becomes lessened and the product temperature rises until eventually it is sufficiently high for the HCO to melt and flow over the wheat particles. The process was completed by reducing the inlet air temperature as rapidly as possible to "freeze" or solidify the meltable hydrophobic substance and maintain discrete treated particles.

### Example 5. Batch Mixers

An alternative means to produce HCO treated product was developed using a high energy mixer, for example a Ploughshare^{®} mixer (Gebr. Lödige, Maschinenbau, Germany). It will be recognized by the person skilled in the art that other mixers of this type can be used. Dry phytase concentrate (Quantum™ Phytase 2500D) is placed into the mixing chamber of a Ploughshare mixer. While the chamber is in motion, molten HCO is delivered from a heated pumping system into the chamber via a jet nozzle or jet nozzles. Optionally, the heating jacket of the mixing chamber may be temperature controlled by pumping water at up to 75°C to prolong the molten state of the HCO during the particle treatment process.

After treatment is complete, the batch is allowed to cool to ambient temperature while maintaining mixing to achieve a free-flowing product similar to those described above.

In a typical process, 8kg of Quantum™ Phytase 2500D is treated with 1600g of molten HCO at approximately 100°C then mixed for 5 minutes. After mixing, the resulting particles have a homogeneous treatment with wax.

### Example 6. Continuous process mixers

Another method was developed using a continuous process mixer, for example the Turbulizer^{®} continuous high shear paddle mixer (Bepex International LLC, Minneapolis, MN). It will be recognized by the person skilled in the art that other mixers of this type can be used. Using a similar process to that described in Example 4, but on a continuous process, Quantum™ Phytase 2500D and molten HCO are fed by metered delivery devices into a mixing chamber. By suitable adjustment of the angles of the paddles in the mixer, the dwell time in the mixer is adjusted to ensure adequate mixing of the components and treatment of the particles with HCO. The resulting free-flowing product obtained is similar to those previously described.

In a typical process, Quantum™ Phytase 2500D was fed by an auger delivery device into the mixing chamber at 205kg/hr. Molten HCO was pumped by a metered heated pump at 41kg/hr. to achieve a free-flowing product similar to those described above.

### Example 7. Pelleting Trials

Mash samples comprising 200 g phytase concentrate/1000kg mash were prepared using HCO-treated and non-treated (QP2500D) phytase particulate material. The mash was pelleted at various controlled temperatures generally in the range of 80°C to 95°C. Samples of the starting mash and the finished pellets were analyzed and the percent recovery of the enzyme in the pelleted product was compared to that in the mash. Five pelleting trials and bioavailability trials were carried out. One storage trial was carried out. Samples were prepared with loadings of HCO on QP2500D of 5%, 7.5%, 10% and 20% by weight.

Results of the pelleting temperature trials are shown in Table 2 for 20% HCO treated and untreated QP2500D. In all pelleting trials conducted, the HCO treated material showed significant enhancement of the numerical recovery of phytase in the feed pellets when compared with the QP2500D standard product (non-HCO treated product).

**Table 2. Percent recovery of phytase activity at different pelleting temperatures.**

| **Treatment** | **% Phytase Recovery at Temperature/ % enhancement over QP2500D** | | |
|---|---|---|---|
| | **85°C** | **88°C** | **90°C** |
| 20% HCO-treated | 92/30 | 79/36 | 66/47 |
| QP2500D | 71/0 | 58/0 | 45/0 |

Table 3 indicates the percent recovery of enzyme activity following pelleting of mash at different temperatures and HCO loads. In general, all HCO concentrations were better than the non-HCO-treated QP2500D control. The data show that there was some improved enzyme protection at HCO concentrations as low as 5% by weight. The improvement increased with concentrations of at least 7.5-20% by weight.

**Table 3. Recovery of enzyme activity after pelleting.**

| Average pelleting temperature (°C) | % Recovery (relative to unprocessed mash)/% enhancement over QP2500D | | | | |
|---|---|---|---|---|---|
| | QP2500D | 5% HCO | 7.5% HCO | 10% HCO | 20% HCO |
| 87.9 | 58/0 | | | | |
| 91.3 | 42/0 | | | | |
| 93.4 | 28/0 | | | | |
| 88.5 | | 63/>09 | | | |
| 91.2 | | 52/24 | | | |
| 93.6 | | 40/>43 | | | |
| 88.5 | | | 74/>28 | | |
| 91.2 | | | 61/45 | | |
| 93.4 | | | 45/61 | | |
| 88.6 | | | | 83/>43 | |
| 91.5 | | | | 58/>38 | |
| 93.1 | | | | 50/»19* | |
| 88.3 | | | | | 76/>31 |
| 91.8 | | | | | 66/>57 |
| 93.0 | | | | | 48/»14* |

The enhancement figures were calculated from the ratio of the recovery relative to the equivalent pelleting temperatures (if within 0.1 °C or less, otherwise, the next lower temperature was used) for QP2500D
* Here, the QP2500D temperature used as reference was significantly below the sample pelleting temperature
Results of the bioavailability trials are shown in Table 4. In all bioavailability trials there was as good phytase bioavailability in the HCO-treated product as in QP2500D and in several of the trials there was better numerical bioavailability in the HCO-treated formulation.

**Table 4. Bioavailability comparison between HCO-treated and non-treated phytase.**

| **Trial Number** | **Animal Body Weight (g)** | |
|---|---|---|
| | **Q2500D** | **20% HCO-treated phytase** |
| 1 | 832 | 830 |
| 2 | 649 | 660 |
| 3 | 645 | 670 |
| 4 | 677 | 729 |
| 5 | 627 | 706 |

Results of the storage trial showed that after at least 3 months of storage the HCO-treated sample to be as good as or better than QP2500D in retention of phytase activity at 21°C.

### Example 8. Extrusion with HCO.

One process typically used for the preparation of solid enzyme-containing products is a granule extrusion process wherein all components are combined in a mixing process with a suitable amount of water to act as both a fluidizing and mixing agent for the components. The resulting wet mixture is then extruded through a suitable extrusion apparatus to produce wet granulates. These wet granulates are then further processed to shape the granules, e.g. cutting to appropriate lengths or spheronising, and then dried to a suitable moisture content.

To test whether the methods of the present invention were applicable to the extrusion process several experiments were carried out with various meltable hydrophobic substances.

For some samples, 50.6 g of Quantum™ Phytase 25000CL (QP25000CL) was mixed with approximately 450 g wheat flour. While these two components were mixing, 46.9 g of molten HCO was poured into the mixture. Approximately 80 g of water was then added and the entire premix was blended for an additional approximately 30 seconds. After this time the premix was extruded through a 0.8 mm screen and the wet strands were broken up and then rounded in spheroniser. It will be recognized that although the product is usually described as spherical, many times the product is "rounded" but not spherical. All such product shapes are included. The 0.8 mm extrudate produces approximately 0.8 mm spheres. It will be recognized by the skilled person in the art that the described method can be used to produce other sized spheres and shapes, for example those with diameters ranging in size from 0.5 to 10 mm. After the spheronising process, the wet granules were transferred to a fluid bed dryer and dried for 20 minutes at approximately 55°C. The dried product was sieved through 1.18 mm and 0.425 mm sieves. The fraction retained on the 0.425 mm sieve was designated as finished product..

Other samples were prepared which contained varying ratios of wax to wheat flour. In the first sample (7% HCO), 50.4 g of Phytase 25000CL (QP25000CL) was blended with approximately 462.9 wheat flour. While these two components were mixing, 34.2 of molten HCO was poured into the mixture. Approximately 100 g of water was then added and the entire premix was blended for an additional approximately 30 seconds. This paste was extruded, spheronised, dried and sieved in a similar fashion to the earlier examples.

In another sample, (13% HCO) 52.7 g of Phytase 25000CL (QP25000CL) was blended with approximately 437.8g wheat flour. While these two components were mixing, 56.3g of molten HCO was poured into the mixture. Approximately 70 g of water was then added and the entire premix was blended for an additional approximately 30 seconds. This paste was extruded, spheronised, dried and sieved in a similar fashion to the earlier examples.

### Example 9. Extrusion with PB3.

For these experiments, 50.6 g QP25000CL was mixed with approximately 450 g of wheat flour. While these two components were mixing, 44.8 g of PB3 was added as a soft solid and blended into the wheat flour. Approximately 70 g of water was then added and the entire premix was blended for an additional approximately 30 seconds. After this time the premix was extruded through a 0.8 mm screen and the wet strands were broken up and then rounded in a spheroniser. The 0.8 mm extrudate produces approximately 0.8 mm spheres. It will be recognized by the skilled person in the art that the described method can be used to produce other sized spheres, for example those with diameters ranging in size from 0.5 to 10 mm. After the spheronising process, the wet granules were transferred to a fluid bed dryer and dried for 20 minutes at approximately 55°C. The dried product was sieved through 1.18 mm and 0.6 mm sieves. The fraction retained on the 0.6 mm sieve was designated as finished product.

### Example 10. Extrusion with other meltable hydrophobic substances.

In further experiments a series of extruded granules were prepared which contained additional examples of meltable hydrophobic substances. These granules were prepared in a similar fashion as described above in that the wax was melted prior to addition to the wheat flour/phytase mixture. The waxes employed are included in Table 1.

### Examples 11. Incorporation of a solid HCO by an extrusion process.

Instead of adding molten HCO or softened PB3, a method was designed to incorporate the solid form of a meltable hydrophobic substance into the extrusion process. Approximately 45 g of HCO flakes was mixed with approximately 450 g of wheat flour. Approximately 51 g of QP25000CL was then added with mixing. Approximately 80 g of water was added and the entire premix was blended for an additional approximately 30 seconds. After this time the premix was extruded through 0.8 mm screen and the wet strands were broken up then rounded in a spheroniser. The wet granules were then transferred to a fluid bed dryer and were dried for 20 minutes at 55°C. The dried product was sieved through 1.18 mm and 0.500 mm sieves. The granules retained by the 0.500 mm sieve were designated as finished product.

### Example 12. Incorporation of solid Akoflake FSR by an extrusion process.

In another experiment, Akoflake FSR, a fully hardened rapeseed oil, was added as a flake and mixed with approximately 450 g of wheat flour. Approximately 51 g of QP25000CL was then added with mixing. Approximately 90 g of water was added and the entire premix was blended for an additional approximately 30 seconds. After this time the premix was extruded through 0.8 mm screen and the wet strands were broken up then rounded in a spheroniser. The wet granules were then transferred to a fluid bed dryer and were dried for 20 minutes at 55°C. The dried product was sieved through 1.18 mm and 0.425 mm sieves. The granules retained by the 0.425 mm sieve were designated as finished product.

### Example 13. Pelleting trials

The granules produced in Examples 8-12 were used to manufacture animal feed through a pelleting process as described in Example 7.

Results of some of the pelleting trials using a molten liquid form (melt) of the meltable hydrophobic substance are shown in Table 5. The results demonstrate that the pellets comprising a wax-infused granule had a higher percent phytase recovery when compared to the QP2500D commercial product, the % recovery being dependent on the wax used. The extrusion process described in Examples 8 to 12 resulted in a free-flowing product that exhibited an increased degree of enzyme protection over the HCO-treated granules described in Examples 2 to 7 above.

**Table 5. Recovery of enzyme activity after pelleting.**

| Average pelleting temp. (°C) | % Recovery (relative to unprocessed mash)/%enhancement over QP2500D | | | | |
|---|---|---|---|---|---|
| | QP2500D | HCO | PB3 | AP | AFM |
| 89.0 | 50/0 | | | | |
| 90.0 | 44/0 | | | | |
| 91.5 | 35/0 | | | | |
| 93.0 | 24/0 | | | | |
| 89.7 | | 96/>92 | | | |
| 91.8 | | 91/>160 | | | |
| 93.6 | | 76>216 | | | |
| 89.5 | | | 89/>78 | | |
| 92.0 | | | 83/>137 | | |
| 93.9 | | | 70>191 | | |
| 89.7 | | | | 84/>68 | |
| 91.8 | | | | 73/>108 | |
| 93.4 | | | | 63/>162 | |
| 89.8 | | | | | 79/>58 |
| 91.8 | | | | | 69/>97 |
| 93.4 | | | | | 63>162 |
| 89.5 | | | | | |
| 91.7 | | | | | |
| 93.5 | | | | | |

The enhancement figures were calculated by the method described for Table 3.

Table 6 shows the relative effect on the percent recoveries achieved for pellets produced using hydrogenated castor oil (HCO) and hydrogenated rapeseed oil (AFx) when added as a molten liquid (melt) or as a solid (flakes). These data demonstrate that the meltable hydrophobic substance may be added as a molten liquid or as a solid without affecting the recovery of enzymatic activity.

**Table 6. Comparison of recovery of enzyme activity using a meltable hydrophobic substance in two different forms, molten liquid and solid.**

| | Ratio % recovery (molten liquid)/% recovery (solid) | | | | | |
|---|---|---|---|---|---|---|
| | Hydrogenated castor oil^{a} | | | Hydrogenated rapeseed oil^{b} | | |
| Temp (°C) | 89.5 | 91.6 | 93.3 | 89.8 | 91.9 | 94.1 |
| Ratio | 1.13 | 0.99 | 1.08 | 0.98 | 0.95 | 1.04 |

| | | | | | | |
|---|---|---|---|---|---|---|
| ^{a} % recovery HCO (melt) Example 8/% recovery HCO (flake) Example 11 ^{b} % recovery AFM (melt) Example 10/% recovery AFF (flake) Example 12 | | | | | | |

## Claims

1. A method for preparing a thermostable, enzyme product for use in the manufacture of animal feed comprising:
a. combining (i) an enzyme, (ii) a solid carrier and (iii) a meltable hydrophobic substance selected from hydrogenated castor oil, a blend of hydrogenated and unhydro-genated vegetable oil, a microcrystalline wax, 12-hydroxystearic acid, hydrogenated palm kernel oil, hydrogenated palm oil, hydrogenated rapeseed oil, and high melting paraffin wax to provide a combined product;
b. optionally applying sufficient heat to the combined product of step (a) to allow the meltable hydrophobic substance to melt;
c. cooling the combined product to provide the thermostable enzyme product; and
d. optionally drying the product to a desired moisture content.

2. The method according to claim 1, wherein in step (a) additional water is added to form a suitable paste mixture and which further comprises the step of extruding the product of step (b) and drying and cooling the extruded product.

3. The method according to any one of claims 1 or 2, wherein a molten form of the meltable hydrophobic substance is added at step (a).

4. The method according to any one, of claims 1 to 3, wherein the enzyme containing solution is first combined with the solid carrier and then the resulting enzyme/carrier combination is combined with the meltable hydrophobic substance.

5. The method according to any one of claims 1 to 4, wherein the meltable hydrophobic substance has a melting temperature of about 25-120°C, preferably about 30-90°C, more preferably about 38-46°C, more preferably about 58-60°C, more preferably about 66-69°C and more preferably about 82-86°C.

6. The method according to any one of claim 1 to 5, wherein the thermostable enzyme product comprises at least 1-30% by weight, preferably at least 5% by weight, preferably at least 7.5% by weight, more preferably at least 10% by weight and most preferably at least 20% by weight of the meltable hydrophobic substance.

7. The method according to any one of claims 1 to 6, wherein the solid carrier is a ground seed grain or mineral, and is preferably selected from the group consisting of ground corn, ground wheat, wheat middlings, soybean meal, rice hulls, corn gluten feed, corn grits, corn flour, wheat flour, distiller's dried grains, diatomaceous earth, silica, and clay.

8. The method according to any one of claims 1 to 7, wherein the enzyme is selected from the group consisting of phytase, phosphatase, xylanase, cellulase, glucanase, mannanase, amylase, alpha-amylase, glucoamylase, peptidase, lipase, esterase, mannase, chitinase, ß-1,3-glucanase, ß-1,4-glucanase, glucose oxidase, catalase, galactosidase, glucosidase, hemicellulase, invertase, lactase, pectinase, pullulase and a protease.

9. The method according to claim 8, wherein the enzyme is a phytase, preferably a thermotolerant phytase and more preferably a thermotolerant phytase comprising SEQ ID NO.1.

10. Use of a thermostable enzyme product obtainable by a method according to any one of claims 1 to 9 together with suitable animal feed ingredients for preparing an animal feed.

## Patentansprüche

1. Verfahren zur Herstellung eines wärmestabilen Enzymproduktes zur Verwendung in der Herstellung von Tierfutter, umfassend:
a. Kombinieren (i) eines Enzyms, (ii) eines festen Trägers und (iii) einer schmelzbaren hydrophoben Substanz, ausgewählt aus hydriertem Rizinusöl, einer Mischung von hydriertem und unhydriertem Pflanzenöl, einem mikrokristallinen Wachs, 12-Hydroxystearinsäure, hydriertem Palmkernöl, hydriertem Palmöl, hydriertem Rapssamenöl und hochschmelzendem Paraffinwachs, unter Bereitstellen eines kombinierten Produktes;
b. gegebenenfalls Anwenden von ausreichend Wärme auf das kombinierte Produkt von Schritt (a), um die schmelzbare hydrophobe Substanz schmelzen zu lassen;
c. Kühlen des kombinierten Produktes unter Bereitstellen des wärmestabilen Enzymproduktes und
d. gegebenenfalls Trocknen des Produktes zu einem gewünschten Feuchtigkeitsgehalt.

2. Verfahren gemäß Anspruch 1, wobei in Schritt (a) zusätzliches Wasser zugesetzt wird, um ein geeignetes Pastengemisch zu bilden, und das außerdem den Schritt des Extrudierens des Produktes von Schritt (b) und Trocknen und Kühlen des extrudierten Produktes umfasst.

3. Verfahren gemäß einem der Ansprüche 1 oder 2, wobei eine geschmolzene Form der schmelzbaren hydrophoben Substanz in Schritt (a) zugesetzt wird.

4. Verfahren gemäß einem der Ansprüche 1 bis 3, wobei die Enzym enthaltende Lösung zuerst mit dem festen Träger kombiniert wird und danach die resultierende Enzym/Träger-Kombination mit der schmelzbaren hydrophoben Substanz kombiniert wird.

5. Verfahren gemäß einem der Ansprüche 1 bis 4, wobei die schmelzbare hydrophobe Substanz eine Schmelztemperatur von etwa 25-120°C, vorzugsweise etwa 30-90°C, bevorzugter etwa 38-46°C, bevorzugter etwa 58-60°C, bevorzugter etwa 66-69°C und bevorzugter etwa 82-86°C hat.

6. Verfahren gemäß einem der Ansprüche 1 bis 5, wobei das wärmestabile Enzymprodukt wenigstens 1-30 Gew.-%, vorzugsweise wenigstens 5 Gew.-%, vorzugsweise wenigstens 7,5 Ges.-%, bevorzugter wenigstens 10 Ges.-% und am bevorzugtesten wenigstens 20 Gew.-% der schmelzbaren hydrophoben Substanz umfasst.

7. Verfahren gemäß einem der Ansprüche 1 bis 6, wobei der feste Träger gemahlene Samenkörner oder gemahlenes Mineral ist und vorzugsweise aus der Gruppe, bestehend aus gemahlenem Mais, gemahlenem Weizen, mittelfeinen Weizenmehlen, Sojabohnenmehl, Reisschalen, Korn- bzw. Mais-Glutenfutter, Korn- bzw. Mais-Grits, Korn- bzw. Maismehl, Weizenmehl, getrocknetem Trester, Diatomeenerde, Siliciumdioxid und Ton, ausgewählt wird.

8. Verfahren gemäß einem der Ansprüche 1 bis 7, wobei das Enzym aus der Gruppe, bestehend aus Phytase, Phosphatase, Xylanase, Cellulase, Glucanase, Mannase, Amylase, alpha-Amylase, Glucoamylase, Peptidase, Lipase, Esterase, Mannase, Chitinase, β-1,3-Glucanase, β-1,4-Glucanase, Glucoseoxidase, Katalase, Galactosidase, Glucosidase, Hemicellulase, Invertase, Lactase, Pectinase, Pullulase und einer Protease, ausgewählt wird.

9. Verfahren gemäß Anspruch 8, wobei das Enzym eine Phytase, vorzugsweise eine wärmetolerante Phytase und bevorzugter eine wärmetolerante Phytase, die SEQ ID NO:1 umfasst, ist.

10. Verwendung eines wärmestabilen Enzymproduktes, erhältlich durch ein Verfahren gemäß einem der Ansprüche 1 bis 9, zusammen mit geeigneten Tierfutteringredienzien zur Herstellung eines Tierfutters.

## Revendications

1. Procédé de préparation d'un produit enzymatique thermostable pour son utilisation dans la fabrication d'un aliment pour animaux comprenant :
a. la combinaison (i) d'une enzyme, (ii) d'un vecteur solide et (iii) d'une substance hydrophobe fusible sélectionnée parmi l'huile de ricin hydrogénée, un mélange d'huile végétale hydrogénée et non hydrogénée, une cire microcristalline, l'acide 12-hydroxystéarique, l'huile de palmiste hydrogénée, l'huile de palme hydrogénée, l'huile de colza hydrogénée, et une cire de paraffine à point de fusion élevé pour fournir un produit combiné ;
b. facultativement l'application d'une chaleur suffisante au produit combiné de l'étape (a) pour permettre la fusion de la substance hydrophobe fusible ;
c. le refroidissement du produit combiné pour fournir le produit enzymatique thermostable ; et
d. facultativement le séchage du produit jusqu'à obtenir une teneur en eau souhaitée.

2. Procédé selon la revendication 1, dans lequel à l'étape (a) de l'eau supplémentaire est ajoutée pour former un mélange pâteux approprié et qui comprend en outre l'étape d'extrusion du produit de l'étape (b) et de séchage et de refroidissement du produit extrudé.

3. Procédé selon l'une quelconque des revendications 1 et 2, dans lequel une forme fondue de la substance hydrophobe fusible est ajoutée à l'étape (a).

4. Procédé selon l'une quelconque des revendications 1 à 3, dans lequel la solution contenant l'enzyme est d'abord combinée au vecteur solide puis la combinaison enzyme/vecteur obtenue est combinée à la substance hydrophobe fusible.

5. Procédé selon l'une quelconque des revendications 1 à 4, dans lequel la substance hydrophobe fusible a une température de fusion d'environ 25 à 120 °C, de préférence d'environ 30 à 90 °C, de manière davantage préférée d'environ 38 à 46 °C, de manière davantage préférée d'environ 58 à 60 °C, de manière davantage préférée d'environ 66 à 69 °C et de manière davantage préférée d'environ 82 à 86 °C.

6. Procédé selon l'une quelconque des revendications 1 à 5, dans lequel le produit enzymatique thermostable comprend au moins 1 à 30 % en poids, de préférence au moins 5 % en poids, de préférence au moins 7,5 % % en poids, de manière davantage préférée au moins 10 % en poids, et idéalement au moins 20 % en poids de la substance hydrophobe fusible.

7. Procédé selon l'une quelconque des revendications 1 à 6, dans lequel le vecteur solide est une semence broyée ou un minéral, et est de préférence sélectionné dans le groupe constitué du maïs broyé, du blé broyé, des remoulages, de la farine de soja, des balles de riz, du gluten de maïs, du gruau de maïs, de la farine de maïs, de la farine de blé, des drêches de distillerie, de la terre de diatomées, de la silice, et de l'argile.

8. Procédé selon l'une quelconque des revendications 1 à 7, dans lequel l'enzyme est sélectionnée dans le groupe constitué d'une phytase, d'une phosphatase, d'une xylanase, d'une cellulase, d'une glucanase, d'une mannanase, d'une amylase, d'une alpha-amylase, d'une glucoamylase, d'une peptidase, d'une lipase, d'une estérase, d'une mannase, d'une chitinase, d'une β-1,3-glucanase, d'une β-1,4-glucanase, d'une glucose oxydase, d'une catalase, d'une galactosidase, d'une glucosidase, d'une hémicellulase, d'une invertase, d'une lactase, d'une pectinase, d'une pullulase et d'une protéase.

9. Procédé selon la revendication 8, dans lequel l'enzyme est une phytase, de préférence une phytase thermotolérante et de manière davantage préférée une phytase thermotolérante comprenant SEQ ID NO : 1.

10. Utilisation d'un produit enzymatique thermostable pouvant être obtenu par un procédé selon l'une quelconque des revendications 1 à 9, conjointement à des ingrédients alimentaires pour animaux appropriés, pour la préparation d'un aliment pour animaux.
